# EUROPEAN PATENT APPLICATION

(11) **EP 2 000 173 A2**
(43) Date of publication of application: **10.12.2008**
(21) Application number: 07740251.9
(22) Date of filing: 29.03.2007
(51) Int. Cl.: A61N 1/30

(54) **IONTOPHORETIC APPARATUS**

(30) Priority: 29.03.2006 JP 2006092152
(71) Applicant: TTI ELLEBEAU, INC., Tokyo 140-0002 (JP)
(72) Inventor: AKIYAMA, Hidero, Shinagawa-ku, Tokyo 140-0002 (JP)
(74) Representative: Emde, Eric
(86) International application number: PCT/JP2007/056813
(87) International publication number: WO 2007/111368

(57) **Abstract**

Provided is an iontophoresis device including a non-working electrode assembly (40), a working electrode assembly (20) used to administer a drug ion by iontophoresis, a DC electric power source (12) connected to the non-working electrode assembly and the working electrode assembly with different polarities, in which the working electrode assembly includes at least working electrode (22) connected to the same polarity as that of the drug ions on the direct current power source and drug solution holding portions (28,32) electrically connected to this working electrode and holding a drug serving as the drug ions, the non-working electrode assembly includes at least a non-working electrode connected to a polarity opposite to that of the drug ions on the DC electric power source and an electrolyte solution holding portion electrically connected to the non-working electrode and holding an electrolytic solution, and at least one of the working electrode and the non-working electrode contains any one of a fibrous or net-like metal and chloride thereof. With the arrangement, the iontophoresis device can be used for a long time period.

## Description

### Technical Field

The present invention relates to an iontophoresis device for administering a drug ion to an living body by applying a voltage, in particular, an iontophoresis device that can be used for a long time period.

### Background Art

An iontophoresis device for administering a drug ion to an living body by applying a voltage is, for example, one described in WO 03037425 (hereinafter referred to as "Patent Document 1"). The iontophoresis device described in Patent Document 1 has a working electrode assembly and a non-working electrode assembly, and the drug ion is administered from an ion exchange membrane of the working electrode assembly to the skin or mucosa of the living body.

The working electrode assembly in the iontophoresis device described in Patent Document 1 is constituted by laminating, from its side in contact with the skin or mucosa, a first ion exchange membrane, a drug solution holding portion, a second ion exchange membrane, a first electrolyte solution holding portion, and a working electrode in the stated order.

In addition, the non-working electrode assembly is constituted by laminating, from its side in contact with the skin or mucosa, a fourth ion exchange membrane, a third electrolyte solution holding portion, a third ion exchange membrane, a second electrolyte solution holding portion, and a non-working electrode in the stated order.

In the iontophoresis device described in Patent Document 1, the composition of the electrolyte solution can be selected independent of the drug because the drug solution in the drug solution holding portion and the electrolyte solution are partitioned by the second ion exchange membrane.

Therefore, an electrolyte solution that does not generate any chlorine ion by energization can be used, and the selection of an electrolyte having a lower oxidation or reduction potential than a voltage needed for the electrolysis of water as an electrolyte in the electrolyte solution can suppress the production of an oxygen gas, hydrogen gas, hydrogen ion, or hydroxyl ion resulting from the electrolysis of water. Alternatively, the use of a buffer electrolyte solution in which multiple kinds of electrolytes are dissolved can suppress a change in its pH due to the production of a hydrogen ion or hydroxyl ion. Further, in the iontophoresis device, the transfer of the drug ion to the first electrolyte solution holding portion is blocked by the second ion exchange membrane, so the following problem is solved: the drug alters owing to a chemical reaction at the time of the energization.

Meanwhile, it has been requested that, for example, insulin for patients suffering from diabetes be administered little by little over a long time period. In contrast, the time period for which energization is performed in an iontophoresis device using an electrode solution may be prolonged depending on the amount or concentration of the electrode solution. On the other hand, in the case where, for example, a silver electrode is used because of chlorine contained in a drug, no electrode solution is needed, a metal plate is used, and whether the time period for which energization is performed on the electrode is long depends on the surface area of the electrode; however, no particular contrivance to lengthen the time period has been made.

### Disclosure of the Invention

An object of the present invention is to provide an iontophoresis device which can be used for a long time period by using a special electrode.

The present invention provides an iontophoresis device including a working electrode assembly and a non-working electrode assembly which are used for administering a drug ion by means of iontophoresis; and a DC electric power source to be connected to the working electrode assembly and the non-working electrode assembly with different polarities, respectively, in which: the working electrode assembly includes at least: a working electrode connected to the same polarity as that of the drug ion in the DC electric power source; and a drug solution holding portion for holding a drug serving as the drug ion, the drug solution holding portion being electrically connected to the working electrode; the non-working electrode assembly includes at least: a non-working electrode connected to a polarity opposite to that of the drug ion in the DC electric power source; and an electrolyte solution holding portion for holding an electrolyte solution, the electrolyte solution holding portion being electrically connected to the non-working electrode; and at least one of the working electrode and the non-working electrode includes any one of a fibrous metal and a chloride thereof, whereby the above-mentioned problems are solved.

Further, at least one of the fibrous metal and the chloride thereof includes a fibrous carrier and one of a metal and a chloride thereof which cover the fibrous carrier.

Further, at least one of the fibrous metal and the chloride thereof includes a fibrous carrier and a nanoparticle of one of a metal and a chloride thereof which is attached on the fibrous carrier.

Further, at least one of the fibrous metal and the chloride thereof combines with one of a sheet-like carrier and a bulk-like carrier.

Further, at least one of the fibrous metal and the chloride includes: one of a sheet-like carrier and a bulk-like carrier; a fibrous carrier combined therewith; and a nanoparticle of one of a metal and a chloride thereof which is attached thereto.

The present invention further provides an iontophoresis device which includes at least one of the working electrode and the non-working electrode including any one of a net-like metal and a chloride thereof, whereby the above-mentioned problems are solved.

Further, at least one of the net-like metal and the chloride thereof includes a net-like carrier and one of a metal and a chloride thereof which covers the net-like carrier.

Further, at least one of the net-like metal and the chloride thereof includes a net-like carrier and a nanoparticle of one of a metal and a chloride thereof which is attached on the net-like carrier.

Further, at least one of the net-like metal and the chloride thereof combines with one of a sheet-like carrier and a bulk-like carrier.

Further, at least one of the net-like metal and the chloride thereof includes: one of a sheet-like carrier and a bulk-like carrier; a net-like carrier combined with one of the sheet-like carrier and the bulk-like carrier; and a nanoparticle of one of a metal and a chloride thereof which is attached to the net-like carrier.

The present invention further provides an iontophoresis device which includes at least one of the working electrode and the non-working electrode including any one of a sheet-like carrier and a bulk-like carrier and a nanoparticle of one of a metal and a chloride thereof which is attached on the carrier, whereby the above-mentioned problems are solved.

According to the present invention, the iontophoresis device can be used for a long time period by enlarging the surface area of an electrode.

### Brief Description of the Drawings

Fig. 1 is an exploded perspective view showing an iontophoresis device according to an embodiment of the present invention.
Fig. 2 is an enlarged plan view showing a working electrode portion and a non-working electrode portion in the iontophoresis device.
Fig. 3 is an enlarged sectional view taken along the line III-III of Fig. 2.
Fig. 4 is an exploded sectional view showing the iontophoresis device.
Fig. 5 is a sectional view showing a state where the iontophoresis device is assembled.
Fig. 6 is a perspective view showing the constitution of an electrode used in a first embodiment of the present invention.
Fig. 7 is a perspective view showing the constitution of an electrode used in a second embodiment of the present invention.
Fig. 8 is a perspective view showing the constitution of an electrode used in a third embodiment of the present invention.
Fig. 9 is a perspective view showing the constitution of an electrode used in a fourth embodiment of the present invention.
Fig. 10 is a perspective view showing the constitution of an electrode used in a fifth embodiment of the present invention.
Fig. 11 is a perspective view showing the constitution of an electrode used in a sixth embodiment of the present invention.
Fig. 12 is a perspective view showing the constitution of an electrode used in a seventh embodiment of the present invention.
Fig. 13 is a perspective view showing the constitution of an electrode used in an eighth embodiment of the present invention. Best Mode for carrying out the Invention

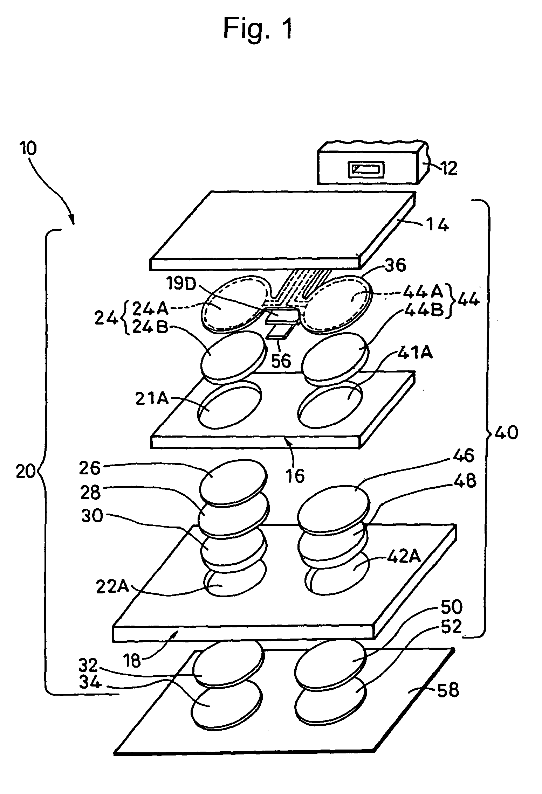

Next, an iontophoresis device 10 according to an exemplary embodiment of the present invention will be described in detail with reference to Figs. 1 to 4.

The iontophoresis device 10 is formed of a DC electric power source 12, a working electrode assembly 20 connected to one of the anode and cathode of the DC electric power source 12, and a non-working electrode assembly 40 connected to the other of the anode and the cathode. The device administers a drug ion held by the working electrode assembly 20 to an living body with a voltage from the DC electric power source 12.

Each of the working electrode assembly 20 and the non-working electrode assembly 40 in the iontophoresis device 10 is constituted by interposing constituent members such as a drug solution holding portion between a base end support 14 and an intermediate support 16 which are overlapped and are each composed of a resin sheet, or by storing the constituent members in through-holes formed in the intermediate support 16 and a tip support portion 18. The base end support 14 and the intermediate support 16 are of the same size, and the tip support 18 is formed so as to be larger than the supports 14 and 16.

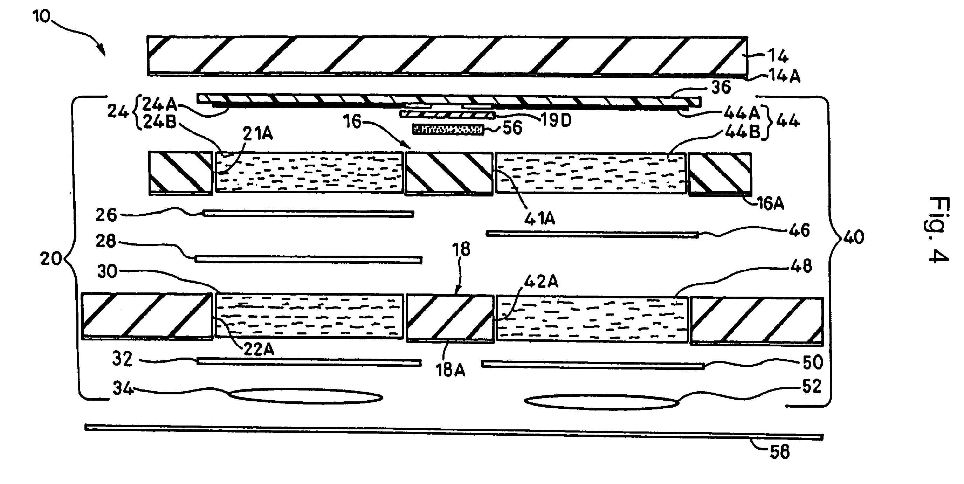

In Fig. 4, the lower surfaces of the base end support 14, the intermediate support 16, and the tip support 18 are provided with a pressure-sensitive adhesive layer 14A, a pressure-sensitive adhesive layer 16A, and a pressure-sensitive adhesive layer 18A, respectively so that the supports are stuck to one another. At the same time, the pressure-sensitive adhesive layer 18A of the tip support 18 is stuck to the skin or mucosa of the living body.

Here, the intermediate support 16 is a single sheet-like member of which part of the working electrode assembly 20 and part of the non-working electrode assembly 40 are formed.

Similarly, the tip support 18 is a single sheet-like member of which part of the working electrode assembly 20 and part of the non-working electrode assembly 40 are formed.

The working electrode assembly 20 is constituted by laminating a working electrode 24 connected to the anode or cathode of the DC electric power source 12, the anode or cathode to which the electrode is connected being of the same polarity as that of the drug ion, a separator 26 placed on the front surface of the working electrode 24, a second ion selective membrane 28 for selectively causing an ion opposite in sign to the drug ion to pass through the membrane, the membrane being placed on the front surface of the separator 26, a drug solution holding portion 30 for holding a drug to serve as the drug ion, the portion being placed on the front surface of the second ion selective membrane 28, a first ion selective membrane 32 for selectively causing an ion of the same kind as that of the drug ion to pass through the membrane, the membrane being placed on the front surface of the drug solution holding portion 30, and a working living body-contacting portion 34 formed by applying a viscous liquid containing a drug identical to the above drug to the front surface of the first ion selective membrane 32 in the stated order.

The working electrode 24 is formed of: a working collector 24A connected to the DC electric power source 12 and composed of a carbon-printed electrode formed by printing on the front surface of a resin sheet 36; and a working polarizable electrode 24B placed while being electrically connected to the front surface of the working collector 24A.

It should be noted that the phrase "electrically connected" comprehends not only the case where the electrode is directly connected to the front surface but also the case where the electrode is connected to the front surface through an electric conductor such as a conductive adhesive (the same holds true for the following).

The intermediate support 16 is composed of a resin material having a thickness substantially equal to that of the working polarizable electrode 24B, and has a working intermediate through-hole 21A of substantially the same shape as that of an external shape in the plane shape of the working polarizable electrode 24B. The working polarizable electrode 24B is stored in the working intermediate through-hole 21A.

In addition, the tip support 18 is composed of a resin material having a thickness substantially equal to that of the drug solution holding portion 30, and has a working tip through-hole 22A of substantially the same shape as that of an external shape in the plane shape of the working polarizable electrode 24B. The drug solution holding portion 30 is stored in the working tip through-hole 22A.

The non-working electrode assembly 40 is constituted by laminating a non-working electrode 44 connected to the anode or cathode of the DC electric power source 12, the anode or cathode to which the electrode is connected being opposite in polarity to the drug ion, a separator 46 placed on the front surface of the non-working electrode 44 to hold an electrolyte solution, an electrolyte solution holding portion 48 for holding the same electrolyte solution, a third ion selective membrane 50 for selectively causing an ion opposite in sign to the drug ion to pass through the membrane, and a non-working living body-contacting portion 52 formed by applying a viscous liquid containing an electrolyte solution identical to the electrolyte solution held by the electrolyte solution holding portion 48 in the stated order from the side of the base end support 14.

The non-working electrode 44 is formed of: a non-working collector 44A composed of a carbon-printed electrode printed on the front surface of the resin sheet 36 so as to be apart from the working collector 24A of the working electrode 24; and a non-working polarizable electrode 44B provided so as to contact the non-working collector 44A.

The non-working polarizable electrode 44B has a thickness equal to that of the intermediate support 16, and is stored in a non-working intermediate through-hole 41A formed in the intermediate support 16. In addition, the electrolyte solution holding portion 48 has a thickness equal to that of the tip support 18, and is stored in a non-working tip through-hole 42A formed in the tip support 18.

In this example, each of the through-holes 22A, 21A, 41A, and 42A is of a circular shape, and, furthermore, each of the working electrode 24, the separator 26, the second ion selective membrane 28, the drug solution holding portion 30, the first ion selective membrane 32, and the working living body-contacting portion 34 is of a circular membrane or sheet shape.

Similarly, each of the non-working electrode 44, the separator 46, the electrolyte solution holding portion 48, the third ion selective membrane 50, and the non-working living body-contacting portion 52 is of a circular membrane or sheet shape.

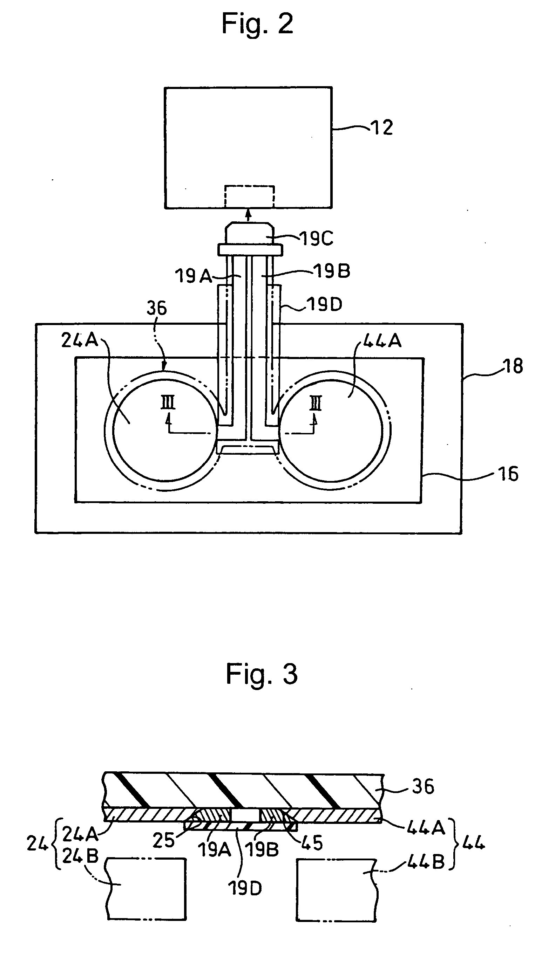

As shown in an enlarged fashion in each of Figs. 2 and 3, a working wire 19A composed of a metal membrane provided to the resin sheet 36 by printing is connected to the working collector 24A in the working electrode 24, and a non-working wire 19B composed of a metal membrane provided to the resin sheet 36 by printing is connected to the non-working collector 44A in the non-working electrode 44.

The tip of each of the working wire 19A and the non-working wire 19B is connected to the DC electric power source 12 through a connector 19C. In addition, an insulating film 19D composed of, for example, a polyimide film is stuck to the surface of each of the working wire 19A and the non-working wire 19B on the side opposite to the resin sheet 36. The insulating film 19D has such a length as to cover the range of each of the working wire 19A and the non-working wire 19B contacting the resin sheet 36 and a certain range of a portion of each wire protruding from the resin sheet 36.

In addition, as shown in an enlarged fashion in Fig. 3, the working wire 19A contacts the working collector 24A as a carbon-printed electrode so as to be connected to the working collector 24A at a tapered surface 25, and the non-working wire 19B contacts the non-working collector 44A as a carbon-printed electrode so as to be connected to the non-working collector 44A at a tapered surface 45.

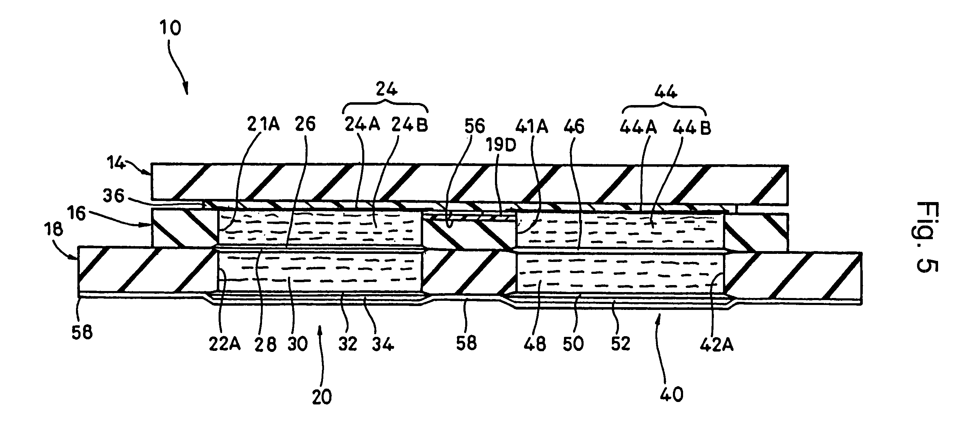

In this embodiment, the respective circular members are overlapped in each of the working electrode assembly 20 and the non-working electrode assembly 40 in the thickness direction of each assembly as shown in each of Figs. 1 and 4, and are integrated as shown in Fig. 5, whereby the iontophoresis device 10 is constituted.

Here, the intermediate support 16 is interposed between other members from above and below the support in a state where the working polarizable electrode 24B and the non-working polarizable electrode 44B are stored in the working intermediate through-hole 21A and non-working intermediate through-hole 41A of the intermediate support 16, respectively. Similarly, the tip support 18 is interposed between other members from above and below the support in a state where the drug solution holding portion 30 and the electrolyte solution holding portion 48 are stored in the working tip through-hole 22A and non-working tip through-hole 42A of the tip support 18, respectively. Further, a member between the base end support 14 and the intermediate support 16 is positioned and fixed by being interposed between them, and a member between the intermediate support 16 and the tip support 18 is positioned and fixed by being interposed between them.

The external diameter of the working collector 24A in the working electrode 24 is slightly larger than the diameter of the working intermediate through-hole 21A so that the working collector 24A can be brought into press contact with the working polarizable electrode 24B. In addition, the external diameter of the non-working collector 44A in the non-working electrode 44 is slightly larger than the diameter of the non-working intermediate through-hole 41A so that the non-working collector 44A can be brought into press contact with the non-working polarizable electrode 44B.

Reference numeral 56 in Fig. 4 represents an adhesive. The adhesive 56 is placed across an intermediate portion between the working and non-working collectors 24A and 44A in the insulating film 19D, and is applied to the back side surface of the intermediate support 16 so that the whole area between the insulating film 19D and the intermediate support 16 is partitioned into a working side and a non-working side.

In addition, reference numeral 58 in Fig. 1 represents a release liner attached to the front surface of the tip support 18 in a peelable fashion so as to cover the working living body-contacting portion 34 and the non-working living body-contacting portion 52.

Next, materials, components, and the like for the above respective constituent elements will be described.

In this embodiment, the drug solution holding portion 30 is constituted by impregnating a polypropylene (PP) non-woven fabric with a viscous liquid containing the drug. In addition, the drug solution holding portion 30 is impregnated with a drug, the drug effect component of which dissociates into a positive or negative ion by, for example, dissolution in a solvent such as water (drug which dissociates into a drug ion (a precursor for the drug is also permitted)). Lidocaine hydrochloride and morphine hydrochloride, respectively an anesthetic and a narcotic, are examples of drugs whose effective component dissociates into positive ions. Ascorbic acid, a vitamin agent, is an example of a drug whose effective component dissociates into negative ions.

In addition to the foregoing, a hormone, DNA, RNA, a protein, an amino acid, and minerals are also included in the category of drugs whose effective component dissociates into positive or negative ions.

The separator 46 in the non-working electrode assembly 40 is obtained by impregnating a PP non-woven fabric with a viscous liquid containing the electrolyte solution (details about the electrolyte solution will be described later). The electrolyte solution holding portion 48 is also obtained by impregnating a PP non-woven fabric with a viscous liquid containing the same electrolyte solution.

The electrolyte solution to be used in each of the separator 46 and the electrolyte solution holding portion 48 is mainly composed of an electrolyte, and electrolytes each of which is oxidized or reduced more readily than the electrolytic reaction of water (oxidation at an anode and reduction at a cathode) such as: pharmaceuticals such as NaCl, ascorbic acid (vitamin C), and sodium ascorbate; and organic acids such as lactic acid, oxalic acid, malic acid, succinic acid, and fumaric acid and/or salts of the acids are each particularly preferably used as the electrolyte. As a result, the generation of an oxygen gas or hydrogen gas can be suppressed. In addition, a fluctuation in pH of the electrolyte solution during energization can be suppressed by blending such a combination of multiple kinds of electrolytes as to provide a buffer electrolyte solution upon dissolution of the above electrolyte in a solvent.

Such viscous liquid containing the drug or electrolyte solution as described above can be produced by, for example, mixing water (ion exchange water) with 2 mass% or more of a tacky material such as hydroxypropylcellulose (HPC) (such as an H-Type manufactured by NIPPON SODA CO., LTD.) or a Metolose as a water-soluble polymer obtained by chemically treating a water-insoluble cellulose (such as a 90SH-10000SR manufactured by Shin-Etsu Chemical Co., Ltd.).

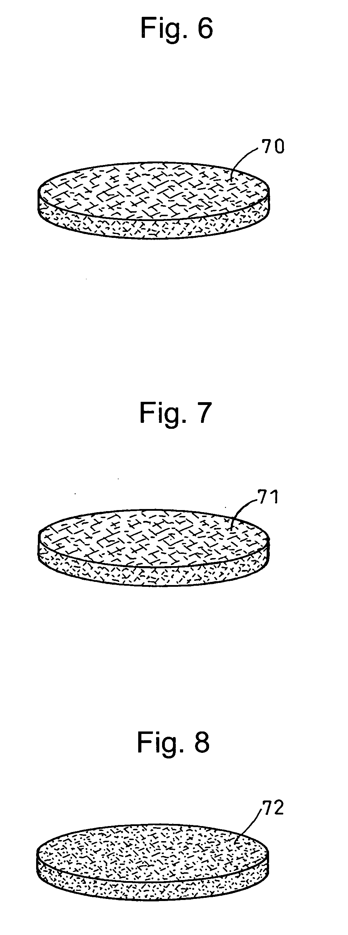

According to a first embodiment of the present invention, as shown in Fig. 6(A), the working polarizable electrode 24B in the working electrode 24 and the non-working polarizable electrode 44B in the non-working electrode 44 are each formed of, for example, silver or silver chloride formed into extra fine fibers 70 each having a diameter of several hundreds of nanometers. Therefore, the surface area of each electrode increases, and hence the iontophoresis device 10 can be used for a long time period.

In this embodiment, each electrode has high durability because the extra fine fibers themselves are each formed of silver or silver chloride.

The working polarizable electrode 24B is impregnated with a viscous liquid containing a drug identical to the drug held by the drug solution holding portion 30. In addition, the non-working polarizable electrode 44B is impregnated with a viscous liquid containing an electrolyte solution identical to the electrolyte solution held by the separator 46.

Each of the working collector 24A in the working electrode 24 and the non-working collector 44A in the non-working electrode 44 is a printed electrode obtained by printing a polyethylene terephthalate (PET) material mixed with carbon and an adhesive.

It should be noted that a conductive metal such as gold, platinum, silver, copper, or zinc as well as carbon may be used as a material for the printed electrode. Alternatively,a conductive material such as carbon or gold itself may be used as a collector without reliance on printing. In addition, a conductive metal such as silver or copper is used as a material for each of the working wire 19A and the non-working wire 19B.

The separator 26 is obtained by impregnating a PP non-woven fabric with a viscous liquid containing a drug identical to the drug held by the drug solution holding portion 30. The separator is interposed between the working polarizable electrode 24B and the second ion selective membrane 28 for preventing energization between them.

The first ion selective membrane 32 is constituted by incorporating an ion exchange resin into which an ion exchange group is introduced so that an ion identical in sign to the drug ion is selectively caused to pass through the resin.

The second ion selective membrane 28 is constituted by incorporating an ion exchange resin into which an ion exchange group is introduced so that an ion opposite in sign to the drug ion is selectively caused to pass through the resin. Here, when a drug effect component in the drug solution holding portion 30 is an anion, a cation exchange resin is blended into the membrane; when the drug effect component is a cation, an anion exchange resin is blended into the membrane.

As in the case of the second ion selective membrane 28, the third ion selective membrane 50 is constituted by incorporating an ion exchange resin into which an ion exchange group is introduced so that an ion opposite in sign to the drug ion is selectively caused to pass through the resin. That is, when the drug solution in the drug solution holding portion 30 dissociates into a cation, the third ion selective membrane 50 contains an anion exchange resin; when the drug solution dissociates into an anion, the membrane contains a cation exchange resin.

An ion exchange resin obtained by introducing a cation exchange group (exchange group the counter ion of which is a cation) such as a sulfonic group, a carboxylic group, or a phosphonic group into a polymer having a three-dimensional network structure such as: a hydrocarbon-based resin such as a polystyrene resin or an acrylic acid-based resin; or a fluorine-based resin having a perfluorocarbon skeleton can be used as the above cation exchange resin without any limitation.

In addition, an ion exchange resin obtained by introducing a cation exchange group (exchange group the counter ion of which is an anion) such as a primary, secondary, or tertiary amino group, a quaternary ammonium group, a pyridyl group, an imidazole group, a quaternary pyridium group, or a quaternary imidazolium group into the same polymer having a three-dimensional network structure as that in the case of the cation exchange resin can be used as the above anion exchange resin without any limitation.

The working living body-contacting portion 34 is constituted by applying a viscous liquid identical to a liquid with which the drug solution holding portion 30 is impregnated to the front surface of the first ion selective membrane 32. In addition, the non-working living body-contacting portion 52 is constituted by applying a viscous liquid containing an electrolyte solution identical to that used in the electrolyte solution holding portion 48 to the front surface of the third ion selective membrane 50. It should be noted that each of those viscous liquids is desirably applied in an amount as small as 7 µL in order that the spread of each of the applied viscous liquids due to the attachment of the release liner 58 on each of the viscous liquids may be suppressed.

At the time of the assembly of the iontophoresis device 10, in a state shown in Fig. 1 or 4, the respective constituent members are placed like a laminate or stored in through-holes, and the base end support 14, the intermediate support 16, and the tip support 18 are sequentially overlapped on the release liner 58, and are fixed by being stuck to one another with the pressure-sensitive adhesive layers 16A and 14A. Thus, the assembly is completed.

A button cell or a thin cell disclosed in, for example, Japanese Patent Application Laid-Open No. Hei 11-067236, US Patent Publication No. 2004/0185667A1, or US Patent No. 6,855,441 can be used as the DC electric power source 12, and the structure of the source is not limited to that in this embodiment.

Next, an effect of this embodiment will be described.

In the iontophoresis device 10 according to this embodiment, the drug solution holding portion and the electrolyte solution holding portions of each of which part of each of the working electrode assembly and the non-working electrode assembly is formed, or the polarizable electrodes each serving as part of an electrode are stored in the through-holes formed in the intermediate support 16 and the tip support 18 each composed of a single sheet-like material, and they are supported by being interposed between other membranes or members. Simultaneously, such members are positioned and fixed between the intermediate support 16 and each of the tip support 18 and the base end support 14 by being interposed between them, whereby the working electrode assembly 20 and the non-working electrode assembly 40 can be integrally constituted at the same time.

In addition, upon production of the iontophoresis device 10, the polarizable electrodes, the ion selective membranes, the drug solution holding portion, the electrolyte solution holding portions, and the like each having substantially the same shape as that of each of the through-holes formed in the tip support 18 and the intermediate support 6 are merely overlapped. Accordingly, the device can be easily produced, and can be brought into mass production.

In addition, in the iontophoresis device 10, each of the working electrode 24 and the non-working electrode 44 is formed of a collector and a polarizable electrode. Accordingly, the device does not cause an electrode reaction at the time of energization in a conventional iontophoresis device, or can achieve energization to an electrolyte solution or drug in a state where such electrode reaction is reduced. As a result, the generation of a gas such as an oxygen gas, a chlorine gas, or a hydrogen gas, or of an unfavorable ion such as a hydrogen ion, a hydroxyl ion, or a hypochlorite ion can be prevented or reduced.

In addition, in the iontophoresis device 10, the working polarizable electrode 24B and the non-working polarizable electrode 44B are stored in the working and non-working intermediate through-holes 21A and 41A of the intermediate support 16, respectively, so that the electrodes are not compressed in their thickness directions. Accordingly, even when each of those electrodes is constituted by impregnating an activated carbon fiber layer with a viscous liquid containing a drug or electrolyte solution, the drug or electrolyte solution with which any such electrode is impregnated does not leak owing to the compression of the electrode by an external force. Similarly, neither the drug of the drug solution holding portion 30 stored in the working tip through-hole 22A nor the electrolyte solution of the electrolyte solution holding portion 48 stored in the non-working tip through-hole 42A leaks owing to an external force.

In addition, in this embodiment, the working collector 24A of which part of the working electrode 24 is formed and the non-working collector 44A of which part of the non-working electrode 44 is formed are each a carbon-printed electrode, and each involve the following problems: each of the collectors is apt to peel, and has a high resistance. However, as shown in Fig. 3, a portion at which the working collector 24A is connected to the working wire 19A and a portion at which the non-working collector 44A is connected to the non-working wire 19B are the tapered surfaces 25 and 45, respectively, and each of the working wire 19A and the non-working wire 19B is formed of a metal such as silver, so the following advantages are obtained: each of the collectors hardly peels from the resin sheet 36, and has a low resistance, and the resin sheet 36 may be curved.

Since the base end support 14 is not stuck to the intermediate support 16 at the portion of the insulating film 19D, conduction may be established between the working electrode 24 and the non-working electrode 44 owing to the infiltration of the drug or electrolyte solution with which a polarizable electrode is impregnated into a space between the supports. In this embodiment, however, the insulating film 19D is bonded to the upper surface of the intermediate support 16 in each of Figs. 1, 4, and 5 with the adhesive 56 at a position between the working collector 24A and the non-working collector 44A to separate the working electrode 24 and the non-working electrode 44, whereby conduction between the electrodes is prevented.

In addition, the separator 26, which is provided for the above working electrode assembly 20, intends to prevent direct conduction between the working polarizable electrode 24B and the second ion selective membrane 28, and is formed of a PP non-woven fabric impregnated with a viscous liquid containing a drug solution. As a result, no voltage is applied to the second ion selective membrane 28, and the electrical characteristics of the second ion selective membrane 28 are prevented from changing. Of course, the separator is not needed when the direct conduction can be prevented by any other means.

The separator 46 in the non-working electrode assembly 40 is also obtained by impregnating a PP non-woven fabric with a viscous liquid containing an electrolyte solution, and its action and effect are the same as those of the separator 26.

In addition, the separator 46 and the electrolyte solution holding portion 48 may be constituted integrally because they have the following characteristic in common: each of the separator and the electrolyte solution holding portion is constituted by impregnating a PP non-woven fabric with a viscous liquid containing an electrolyte solution.

Here, the electrolyte solution holding layer 48, which typically contains an electrolyte solution for retaining conductivity between the non-working electrode 44 and the skin of an living body, may contain a viscous liquid having a drug ion instead of the electrolyte solution so that the drug ion can be administered from the non-working electrode assembly 40.

In addition, it is not necessary to provide the working living body-contacting portion 34 and the non-working living body-contacting portion 52 as long as each of the first ion selective membrane 32 and the third ion selective membrane 50 can sufficiently contact the skin or mucosa of an living body.

Further, in the above embodiment, the working electrode assembly and the non-working electrode assembly each have such a constitution that the drug solution holding portion is placed in a through-hole formed in each of the tip support, the intermediate support, and the base end support, or a membrane is interposed between two adjacent supports of these supports. However, the present invention is not limited to the constitution, and the following constitution is also permitted: none of the tip support, the intermediate support, and the base end support is used, and the drug solution holding portion, the electrolyte solution holding portions, the ion selective membranes, and the like are overlapped in, for example, a case-like backing material.

Although the working and non-working electrodes, the drug solution holding portion, the ion selective membranes, and the first to fourth through-holes are each of a circular shape, the present invention is not limited to the foregoing, and each of them may be of, for example, a quadrangular or hexagonal shape.

Although the surface of each of the working and non-working wires 19A and 19B on the side opposite to the resin sheet 36 is covered with the insulating film 19D, the present invention is not limited to the foregoing, and is applicable also to the case where the insulating film 19D is not provided. In this case, the adhesive 56 is used for bonding the resin film 36 and the intermediate support 16. In short, the adhesive 56 has only to be an adhesive with which a member on the side of the resin film 36 and the intermediate support 16 are bonded to each other.

Next, a second embodiment of the constitution of an electrode according to the present invention will be described.

In this embodiment, as shown in Fig. 7, a fibrous carrier is provided with a coating 71 formed of silver or silver chloride by, for example, plating.

In this embodiment, the electrode can be constituted at a low cost by reducing the amount in which silver or silver chloride is used and forming an inexpensive fibrous carrier.

Next, a third embodiment of the constitution of an electrode according to the present invention will be described.

In this embodiment, as shown in Fig. 8, nanoparticles 72 formed of silver or silver chloride are caused to adhere to fibrous carrier.

In this embodiment, the electrode can be constituted at a low cost by reducing the amount in which silver or silver chloride is used and forming an inexpensive fibrous carrier.

Next, a fourth embodiment of the constitution of an electrode according to the present invention will be described.

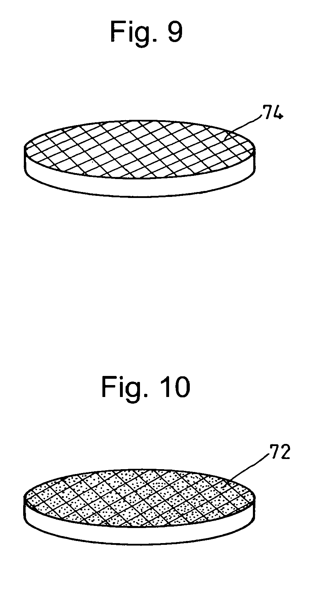

In this embodiment, as shown in Fig. 9, the electrode is silver or silver chloride in the form of a net 74, or is a net-like carrier covered with silver or silver chloride.

In this embodiment, the electrode has high durability because a net is used instead of a fiber.

Next, a fifth embodiment of the constitution of an electrode according to the present invention will be described.

In this embodiment, as shown in Fig. 10, nanoparticles 72 formed of silver or silver are caused to adhere to a net-like carrier.

In this embodiment, the electrode can be constituted at a low cost by forming the fibrous carrier with an inexpensive material and reducing the amount of silver or silver chloride used.

Next, a sixth embodiment of the constitution of an electrode according to the present invention will be described.

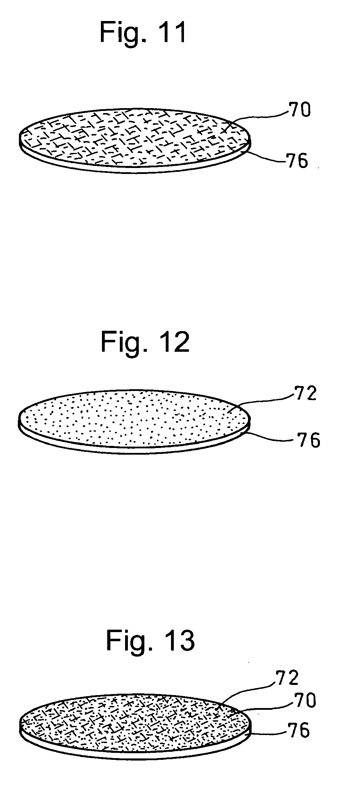

In this embodiment, as shown in Fig. 11, silver or silver chloride shaped into the extra fine fibers 70 is caused to adhere to a sheet-like carrier 76 or bulk-like carrier. It should be noted that the sheet- or bulk-like carrier is placed on the side opposite to the skin of an living body (upper side in each of Figs. 1, 4, and 5) as required.

In this embodiment, the electrode can be constituted at a low cost by forming the sheet- or bulk-like carrier with an inexpensive material and reducing the amount of silver or silver chloride used.

Next, a seventh embodiment of the constitution of an electrode according to the present invention will be described.

In this embodiment, as shown in Fig. 12, nanoparticles 72 formed of silver or silver chloride are caused to adhere to a sheet-like carrier 76 or bulk-like carrier. It should be noted that the sheet- or bulk-like carrier is placed on the side opposite to the skin of an living body (upper side in each of Figs. 1, 4, and 5) as required.

In this embodiment, the electrode can be constituted at a low cost by forming the sheet- or bulk-like carrier with and inexpensive material and reducing the amount of silver or silver chloride used.

Next, a eighth embodiment of the constitution of an electrode according to the present invention will be described.

In this embodiment, as shown in Fig. 13, the sheet-like carrier 76 or bulk-like carrier is combined with a carrier formed of the fibers 70, and, furthermore, nanoparticles 72 each formed of silver or silver chloride are caused to adhere to the resultant. It should be noted that the sheet- or bulk-like carrier is placed on the side opposite to the skin of an living body (upper side in each of Figs. 1, 4, and 5) as required.

In this embodiment, the electrode can be constituted at a low cost by forming the sheet- of bulk like carrier with an inexpensive material and reducing the amount of silver or silver chloride used.

Although silver is used as a metal in each of the embodiments, the kind of the metal is not limited to silver.

Although each of the working electrode 24 and non-working electrode 44 of the above iontophoresis device 10 is an electrode according to the present invention, one of the electrodes may be a capacitor electrode obtained by combining a carbon-printed electrode and an activated carbon layer.

### Industrial Applicability

The present invention can be utilized as an iontophoresis device for administering a drug ion to an living body by applying a voltage, in particular, an iontophoresis device that can be used for a long time period.

## Claims

1. An iontophoresis device, comprising:
a working electrode assembly and a non-working electrode assembly used for administering a drug ion by means of iontophoresis; and
a DC electric power source to be connected to the working electrode assembly and the non-working electrode assembly with different polarities, respectively,
**characterized in that:**
the working electrode assembly includes at least: a working electrode connected to the same polarity as that of the drug ion in the DC electric power source; and a drug solution holding portion for holding a drug serving as the drug ion, the drug solution holding portion being electrically connected to the working electrode;
the non-working electrode assembly includes at least: a non-working electrode connected to a polarity opposite to that of the drug ion in the DC electric power source; and an electrolyte solution holding portion for holding an electrolyte solution, the electrolyte solution holding portion being electrically connected to the non-working electrode; and
at least one of the working electrode and the non-working electrode including any one of a fibrous metal and a chloride thereof.

2. The iontophoresis device according to claim 1, **characterized in that** at least one of the fibrous metal and the chloride thereof comprises a fibrous carrier, and one of a metal and a chloride thereof, covering the fibrous carrier.

3. The iontophoresis device according to claim 1, **characterized in that** at least one of the fibrous metal and the chloride thereof comprises a fibrous carrier, and a nanoparticle of one of a metal and a chloride thereof, attached to the fibrous carrier.

4. The iontophoresis device according to claim 1, **characterized in that** at least one of the fibrous metal and the chloride thereof is combined with one of a sheet-like carrier and a bulk-like carrier.

5. The iontophoresis device according to claim 1, **characterized in that** at least one of the fibrous metal and the chloride thereof comprises:
one of a sheet-like carrier and a bulk-like carrier;
a fibrous carrier combined therewith; and
a nanoparticle of one of a metal and a chloride thereof which is attached thereto.

6. An iontophoresis device, comprising:
a working electrode assembly and a non-working electrode assembly used for administering a drug ion by means of iontophoresis; and
a DC electric power source connected to the working electrode assembly and the non-working electrode assembly with different polarities, respectively,
**characterized in that:**
the working electrode assembly includes at least: a working electrode connected to the same polarity as that of the drug ion in the DC electric power source; and a drug solution holding portion for holding a drug serving as the drug ion, the drug solution holding portion electrically connected to the working electrode;
the non-working electrode assembly includes at least: a non-working electrode connected to a polarity opposite to that of the drug ion in the DC electric power source; and an electrolyte solution holding portion for holding an electrolyte solution, the electrolyte solution holding portion electrically connected to the non-working electrode; and
at least one of the working electrode and the non-working electrode including any one of a net-like metal and a chloride thereof.

7. The iontophoresis device according to claim 6, **characterized in that** at least one of the net-like metal and the chloride thereof comprises a net-like carrier, and one of a metal and a chloride thereof, covering the net-like carrier.

8. The iontophoresis device according to claim 6, **characterized in that** at least one of the net-like metal and the chloride thereof comprises a net-like carrier, and a nanoparticle of one of a metal and a chloride thereof, attached to the net-like carrier.

9. The iontophoresis device according to claim 6, **characterized in that** at least one of the net-like metal and the chloride thereof is combined with one of a sheet-like carrier and a bulk-like carrier.

10. The iontophoresis device according to claim 6, **characterized in that** at least one of the net-like metal and the chloride thereof comprises:
one of a sheet-like carrier and a bulk-like carrier;
a net-like carrier combined with one of the sheet-like carrier and the bulk-like carrier; and
a nanoparticle of one of a metal and a chloride thereof which is attached to the net-like carrier.

11. An iontophoresis device, comprising:
a working electrode assembly and a non-working electrode assembly used for administering a drug ion by means of iontophoresis; and
a DC electric power source connected to the working electrode assembly and the non-working electrode assembly with different polarities, respectively,
**characterized in that:**
the working electrode assembly includes at least: a working electrode connected to the same polarity as that of the drug ion in the DC electric power source; and a drug solution holding portion for holding a drug serving as the drug ion, the drug solution holding portion electrically connected to the working electrode;
the non-working electrode assembly includes at least: a non-working electrode connected to a polarity opposite to that of the drug ion in the DC electric power source; and an electrolyte solution holding portion for holding an electrolyte solution, the electrolyte solution holding portion electrically connected to the non-working electrode; and
at least one of the working electrode and the non-working electrode includes any one of a sheet-like carrier and a bulk-like carrier, and a nanoparticle of one of a metal and a chloride thereof, attached to the carrier.
